(19) Europäisches Patentamt
European Patent Office
Office européen des brevets

(11) Veröffentlichungsnummer: **0 029 909**
**B1**

(12) # EUROPÄISCHE PATENTSCHRIFT

(45) Veröffentlichungstag der Patentschrift: **18.07.84**

(51) Int. Cl.³: **C 07 C 102/04**, C 07 C 103/52, C 07 D 295/12

(21) Anmeldenummer: **80106416.3**

(22) Anmeldetag: **21.10.80**

(54) **Verfahren zur Herstellung von Säureamiden, Verwendung des Verfahrens und Morpholino-äthyl-isocyanid.**

(30) Priorität: **22.10.79 DE 2942606**

(43) Veröffentlichungstag der Anmeldung: **10.06.81 Patentblatt 81/23**

(45) Bekanntmachung des Hinweises auf die Patenterteilung: **18.07.84 Patentblatt 84/29**

(84) Benannte Vertragsstaaten: **AT CH DE FR GB IT LI**

(56) Entgegenhaltungen:
**US - A - 3 933 783**

**TETRAHEDRON LETTERS, Nr. 36, September 1978, Oxford, New York, Paris, Frankfurt H. AIGNER et al. "Synthese von Amiden aus Carbonsäuren und Aminen in Gegenwart von Isocyaniden" Seiten 3325 bis 3326**
**SYNTHESIS, März 1979 L. WACKERLE "Racemisierungsfreie Aktivierung von alpha-Aminosäuren mittels Isocyaniden" Seiten 197 bis 198**

**Die Akte enthält technische Angaben, die nach dem Eingang der Anmeldung eingereicht wurden und die nicht in dieser Patentschrift enthalten sind.**

(73) Patentinhaber: **Marquarding, Bozena, Darmstädter Strasse 11, D-8000 München 50 (DE)**
Patentinhaber: **Aigner, Helmut, Dr., Barerstrasse 52, D-8000 München 40 (DE)**
Patentinhaber: **Marquarding, Marcel, Darmstädter Strasse 11, D-8000 München 50 (DE)**

(72) Erfinder: **Marquarding, Dieter, Dr., Verstorben (DE)**
Erfinder: **Aigner, Helmut, Dr., Barerstrasse 52, D-8000 München 40 (DE)**

(74) Vertreter: **Hansen, Bernd, Dr.rer.nat. et al, Hoffmann, Eitle & Partner Patentanwälte Arabellastrasse 4, D-8000 München 81 (DE)**

EP 0 029 909 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft Verfahren zur Herstellung von Säureamiden durch Umsetzung von Carbonsäure und Amin in Anwesenheit von Isonitril, sowie Anwendungen dieses Verfahrens.

Zahlreiche Verfahren zur Darstellung von Amiden aus Carbonsäuren und Aminen sind bekannt. Besonderes Interesse besitzen Amidsynthesen bei der Darstellung von Peptiden sowie pharmazeutisch wirksamen Systemen, die Amidgruppierungen enthalten.

Im Bereich der Peptidsynthese hat die sogenannte Carbodiimid-Methode für die Knüpfung von Amidbindungen große Bedeutung erlangt. Dieses Verfahren besitzt aber zum Teil schwerwiegende Nachteile. Einmal ist das bei der Umsetzung aus dem Carbodiimid regelmäßig gebildete Harnstoffderivat häufig außerordentlich schwierig vom gewünschten Reaktionsprodukt abzutrennen. Bei einigen dieser Harnstoffderivate wurde auch eine carzerogene Wirkung nachgewiesen. Darüber hinaus treten vielfach unerwünschte Nebenreaktionen ein, die unter anderem zur Bildung von N-Acylharnstoffen führen. Die Abtrennung dieser, die Ausbeute nachteilig beeinflussender Nebenprodukte bringt zudem teilweise schwerwiegende Probleme mit sich.

Durch die Anmelder ist in Tetrahedron Letters, Nr. 36, Seiten 3325 bis 3326 (1978) die Synthese von Amiden aus Carbonsäuren und Aminen in Gegenwart von Isocyaniden beschrieben werden. Auch eine Mitteilung in »Synthesis«, März 1979, Seiten 197 bis 198, beschäftigt sich mit einer solchen Säureamidbildung und deren Anwendung auf die Peptidsynthese. In den vorbeschriebenen Amidierungen wurden als Aktivierungsmittel verschiedene aliphatische und cycloaliphatische Isonitrile verwendet. Die erreichbaren Ausbeuten sind insbesondere dann, wenn es um die Synthese etwas schwieriger zu synthetisierender Peptidsysteme geht, allerdings noch unbefriedigend.

Schließlich ist aus der US-A-3 933 783 ein Verfahren zur Herstellung von Säureamiden durch Umsetzung von Karbonsäure und Amin in Anwesenheit von Ethoxycarbonylmethylisonitril bekannt, das neben anderen Isonitrilen genannt ist. Der einsatz dieser Isonitrile, deren Substituenten gemäß Spalte 2, Zeilen 31 ff, an der Reaktion nicht beteiligt sein sollen, führt im Hinblick auf die Reinheit der anfallenden Produkte, erreichbare Ausbeute und bei schwierig synthetisierbaren Peptidenden Eintritt der Reaktion überhaupt häufig nicht zu akzeptablen Ergebnissen.

Der Erfindung liegt die Aufgabe zugrunde, ein Verfahren zur Amidierung zu schaffen, das in hohen Ausbeuten und racemisierungssicher die Synthese von Amiden, insbesondere Peptiden aber auch anderer Amidsysteme mit pharmazeutischer Wirkung gestattet, wobei die gewünschten Amide auf einfache Weise aus dem Reaktionsgemisch gewonnen werden können. Weiterhin soll durch ein solches Verfahren die Bildung von Amidsystemen, die nach herkömmlichen Verfahren und insbesondere der bekannten Carbodiimidmethode in befriedigender Ausbeute nicht synthetisierbar sind, in hoher Ausbeute erreicht werden.

Diese Aufgabe wird durch ein Verfahren zur Herstellung von Säureamiden durch Umsetzung von Karbonsäure und Amin in Anwesenheit von Isonitril gelöst, das dadurch gekennzeichnet ist, daß ein basisches Isonitril mit mindestens einem N-Atom in dem an der Isonitrilgruppe befindlichen Substituenten eingesetzt wird.

Durch die Verwendung von basischen Isonitrilen mit mindestens einem N-Atom in dem an der Isonitrilgruppe befindlichen Substituenten als aktivierendes und wasserabspaltendes Mittel können Säureamide und insbesondere auch schwierig zugängige Peptidsysteme in überraschend hoher Ausbeute auf einfache Weise gewonnen werden. Dies ist deshalb besonders überraschend, weil sich bei der Herstellung bekannter Peptidsysteme unter Verwendung modifizierter Carbodiimide, z. B. beim Einsatz von Carbodiimiden mit tertiären Aminogruppen, im allgemeinen keine Erhöhung der Ausbeute an Peptid erreichen läßt. So zeigen die herkömmlichen Carbodiimide, die im übrigen mit den Isonitrilen aus verschiedenen Gründen nicht direkt vergleichbar sind, bei Einfügung von Heteroresten keine Neigung zu einer höheren Reaktionsfreude, die insbesondere bei der Synthese schwieriger zugänglicher Peptide wünschenswert wäre. Im übrigen haben auch derartig modifizierte Carbodiimide, teils aus Gründen ihrer schweren Zugänglichkeit, teils aus Gründen anderer Nebenreaktionen, etc., keinen breiteren Eingang in die Praxis gefunden.

Nach einer Arbeitshypothese, auf die hier aber keine Festlegung erfolgen soll, wird durch das freie Elektronenpaar des N-Atoms eine Protonisierung erleichtert, die als Zwischenstufe die Protonierung der Isonitrilfunktionen begünstigen könnte.

Besonders günstige Ergebnisse werden beim erfindungsgemäßen Verfahren mit Isonitrilen erzielt, bei denen das Heteroatom im Substituenten an der Isonitrilgruppe und das C-Atom der funktionellen Isonitrilgruppe sich in 1,5- oder 1,6-Stellung zueinander befinden.

Besonders günstige Ausbeuten erreicht man insbesondere mit N-heterocyclisch-substituierten Isonitrilen. Besonders geeignete, erfindungsgemäß anwendbare Isonitrile sind auch solche, bei denen die Heterobindung elektronenschiebende Gruppen bzw. Substituenten, z. B. Methyl- oder Allylgruppen, enthält.

Die basischen Isonitrile der genannten Art bieten zusätzlich die Möglichkeit der sehr leichten Abtrennung von den als Begleitprodukt gebildeten Formamiden, weshalb solche basischen Isonitrile besonders bevorzugt werden.

Typische Vertreter der für die Amidierung erfindungsgemäß verwendeten Isonitrile sind:

Isonitrile:

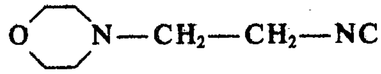

MAI

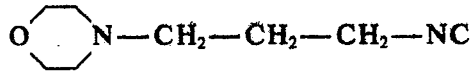

MPI

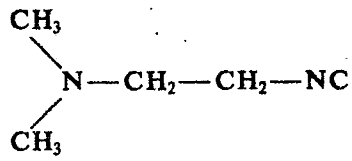

DMAAI

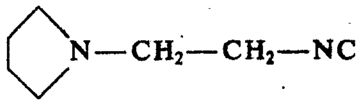

PAI

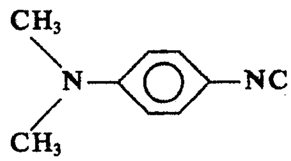

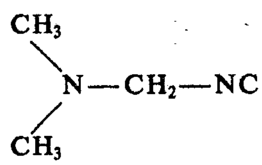

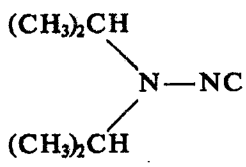

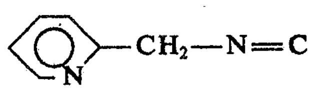

Die meisten der vorstehenden Verbindungen sind literaturbekannt, weshalb ihre Synthese nach den dort angegebenen Verfahren leicht erfolgen kann. Eine Ausnahme hierunter stellt allerdings Morpholino-äthyl-isocyanid (MAI) dar, das ausgehend von 2-(4'-Morpholinyl)-äthyl-amin durch die bei der Herstellung von Isonitrilen bekannte Phosgenmethode erhältlich ist. Diese neue Verbindung, deren Einsatz im erfindungsgemäßen Verfahren besonders bevorzugt ist, hat einen Kp bei 0,05 Torr von 70° C und eine Dichte bei Raumtemperatur von 1,014. Bezüglich der allgemein anwendbaren Synthesemethodik wird verwiesen auf »Angewandte Chemie«, 77. Jg. 1965/Nr. 11.

Der Ablauf des Amidierungsverfahrens gemäß der Erfindung läßt sich durch nachstehendes Reaktionsschema, auf das eine Festlegung hier nicht erfolgen soll, gut erklären:

## Reaktionsschema

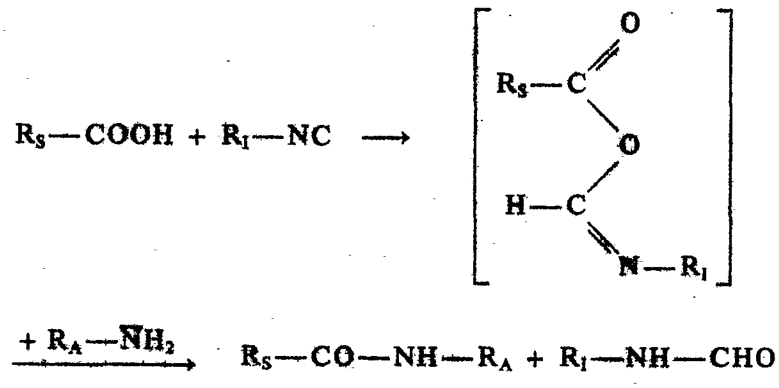

$R_S$ = Säurerest
(bei Peptiden N-terminal geschützter Aminosäure- bzw. Peptidrest)
$R_I$ = Substituent des Isonitrils mit Heteroatom
$R_A$ = Aminrest
(bei Peptiden C-terminal geschützter Aminosäure- bzw. Peptidrest)

Die Herstellung des Amids kann in einem geeigneten protischen oder aprotischen Lösungsmittel durchgeführt werden. Derartige Lösungsmittel sind beispielsweise Isopropanol, Dichlormethan, Chloroform, Tetrachlorkohlenstoff, Äthylacetat, Dimethylformamid etc. Die Reaktion tritt gewöhnlich bereits bei Raumtemperatur ein und ist recht rasch beendet. Lediglich bei schwieriger zugänglichen Systemen, die zum Teil nach der herkömmlichen Carbodiimidmethodik oder bislang üblichen Isonitrilen gar nicht herstellbar sind, war eine Reaktion bei etwas erhöhter Temperatur notwendig.

Das erfindungsgemäße Verfahren hat seine besondere Anwendung bei der schonenden Herstellung von Amidbindungen. Es kann mit besonderem Vorteil zur Herstellung von Peptiden eingesetzt werden. Hierbei kann im allgemeinen nach der nachstehenden Arbeitsvorschrift verfahren werden:

### Arbeitsvorschrift

Zu einer Lösung von 2,5 mmol der Säurekomponente (zusammen mit 5 mmol Hydroxysuccinimid) und 2,5 bis 3 mmol Isonitril in einem geeigneten Lösungsmittel (z. B. Dichlormethan, Essigsäureäthylester, Dioxan, Isopropanol, Dimethylformamid) werden nach 30 Minuten 2,5 mmol der Aminkomponente, gegebenenfalls als Hydrochlorid, zusammen mit 2,5 mmol Triäthylamin, gegeben. Das Reaktionsgemisch wird ca. 4 Stunden, z. B. bei Raumtemperatur, gerührt. Dann engt man im Vakuum ein, nimmt den Rückstand, z. B. in Essigester, auf, schüttelt zweimal mit schwacher Säure, z. B. 1N-Salzsäure oder Zitronensäurelösung, danach zweimal mit (z. B. 7,5%iger) Natriumhydrogencarbonatlösung und schließlich zweimal mit Wasser aus. Die organische Phase wird über Natriumsulfat getrocknet. Das Produkt erhält man nach vollständigem Abdampfen des Lösungsmittels im Vakuum, üblicherweise in analysenreiner Form.

Liegt in der Isonitril-Ausgangskomponente keine basische Funktion vor, wird das entstandene Formamidderivat durch Destillation oder auch Umkristallisation entfernt.

Aus obiger Arbeitsvorschrift ergibt sich, daß es in vielen Fällen günstig — wenngleich auch nicht immer notwendig — ist, der Reaktion zusätzlich 2 mmol N-Hydroxysuccinimid pro mmol Säurekomponente zugegeben. Hierdurch wird, ebenso wie bei der bekannten Carbodiimidsynthese eine hohe Racemisierungssicherheit erreicht.

Nach dieser Arbeitsvorschrift wurden die nachstehenden Amidderivate, die überwiegend Peptide darstellen, hergestellt:

| Produkt | Lösungsmittel | Isonitril | Ausbeute % | Fp. °C | | Analyse C | H | N | Bemerkung |
|---|---|---|---|---|---|---|---|---|---|
| Benzoesäurebenzylamid | $CH_2Cl_2$ | MAI | 69 | 106 | | | | | Reaktionsdauer 3 Tage |
| Z–Val–Gly–OMe | $CH_2Cl_2$ | MAI | 80 | 157–158 | ber. | 59,62 | 6,88 | 8,69 | |
| | | | | | gef. | 59,72 | 6,86 | 8,67 | |
| Z–Val–Gly–OMe | Essigester | MAI | 81 | 156–158 | ber. | 59,62 | 6,88 | 8,69 | |
| | | | | | gef. | 59,58 | 6,93 | 8,69 | |
| Z–Val–Val–OMe | $CH_2Cl_2$ | MAI | 90 | 98–100 | ber. | 62,62 | 7,74 | 7,69 | |
| | | | | | gef. | 62,74 | 7,58 | 7,81 | |
| Z–Val–Val–OtBu | $CH_2Cl_2$ | MAI | 91 | fest | | | | | Drehmoment des Produktes $[\alpha]_D^{20}$ −32,5 c = 0,4 EtOH |
| Z–Leu–Gly–OEt | $CH_2Cl_2$ | MAI | 90 | 98–100 | | | | | |
| Z–Leu–Leu–OMe | $CH_2Cl_2$ | MAI | 94 | 79–81 | | | | | |
| Z–Ala–Phe–OMe | $CH_2Cl_2$ | MAI | 94 | 96–97 | ber. | 65,61 | 6,29 | 7,29 | |
| | | | | | gef. | 65,49 | 6,16 | 7,39 | |
| Z–Ala–Pro–OMe | $CH_2Cl_2$ | MAI | 87 | Öl | ber. | 61,03 | 6,64 | 8,39 | |
| | | | | | gef. | 60,96 | 6,61 | 8,26 | |
| Boc–Aib–Aib–OMe | $CH_2Cl_2$ | MAI | 68 | | | | | | Reaktionsdauer 7 Tage/40°C |
| TFA–Phe–Gly–Ome | $CH_2Cl_2$ | MAI | 77 | 118–121 | | | | | |
| TFA–Phe–Phe–OMe | $CH_2Cl_2$ | MAI | 76 | 174–176 | | | | | |
| Boc–Gly–Ala–Val–OMe*) | $CH_2Cl_2$ | MAI | 90 | 104–106 | ber. | 53,47 | 8,13 | 11,69 | ohne Hosu**) |
| | | | | | gef. | 53,37 | 7,87 | 11,75 | |
| Boc–Gly–Ala–Val–OtBu*) | $CH_2Cl_2$ | MAI | 96 | 106–108 | | | | | |
| Z–Ala–GlyOMe | $CH_2Cl_2$ | DMAAI | 68 | 92–94 | | | | | |
| Z–Val–GlyOMe | $CH_2Cl_2$ | DMAAI | 62 | 155–156 | | | | | |
| Z–Ala–GlyOMe | $CH_2Cl_2$ | PAI | 61 | 91–94 | | | | | |

Fortsetzung

| Produkt | Lösungs-mittel | Isonitril | Aus-beute % | Fp. °C | Analyse | C | H | N | Bemerkung |
|---|---|---|---|---|---|---|---|---|---|
| Z–Val–GlyOMe | $CH_2Cl_2$ | $(CH_3)_2$—N 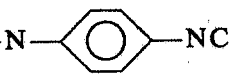 NC | 67 | 150–152 | | | | | umkristallisiert/ ohne Hosu |
| Bz–Val–GlyOMe | Isopropanol | MPI | 80 | | ber.<br>gef. | 63,73<br>63,86 | 7,55<br>7,72 | 8,74<br>8,85 | ohne Hosu/ umkristallisiert |
| Z–Val–GlyOMe | Acetonitril | MPI | 61 | 156–158 | | | | | |

*) Boc–Gly–Ala–OH, als Säurekomponente.
**) Hosu = Hydroxy-succinimid.

Die in der Tabelle angegebenen Begriffe bedeuten:

| | | |
|---|---|---|
| Z | – | Carbobenzoxy |
| Boc | = | t-Butyl-oxycarbonyl |
| TFA | = | Trifluoracetyl |
| Bz | = | Benzoyl |
| Aib | = | $\alpha$-Amino-i-buttersäure. |

Die Identifizierung des gebildeten Reaktionsproduktes erfolgte, soweit keine Werte der Mikroanalyse angegeben sind, durch übliche spektroskopische Methodik. Die angegebenen Ausbeutewerte sind noch nicht optimiert und daher gegenüber den teilweise recht hohen Werten noch zusätzlich steigerungsfähig. Aus der Tabelle ergibt sich, daß auch schwerer zugängliche Peptidsysteme in hoher Ausbeute erhalten werden können, die z. B. unter Anwendung des aus der US-A-3 933 783 bekannten $CN-CH_2COOEt$ nicht zugängig sind.

Darstellung MAI

1. Stufe

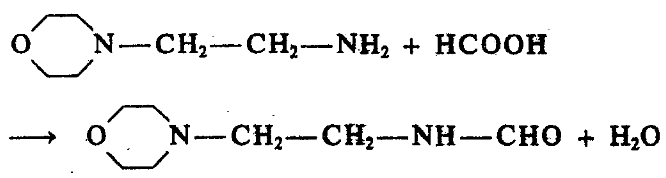

0,7 Mol Amin und 30 ml Ameisensäure in 300 ml Toluol werden am Wasserabscheider zum Sieden erhitzt. Wenn das Reaktionswasser abdestilliert ist, wird im Vakuum eingeengt und anschließend in Dichlormethan aufgenommen. In die Lösung leitet man bis zur Sättigung Ammoniak ein, filtriert und engt erneut im Vakuum ein. Den Rückstand destilliert man im Hochvakuum.

Kp. 110° C 0,05 Torr
H–NMR: –CHO ppm ($\delta$) = 8,0 (s) TMS als innerer Standard
IR: C=O 1670 $cm^{-1}$

2. Stufe

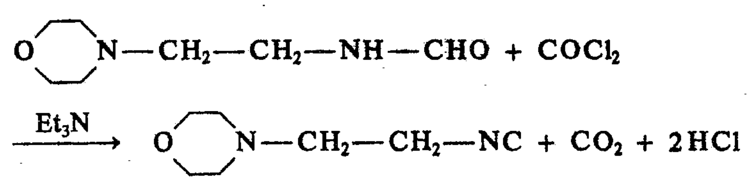


1 Mol 2-Morpholino-äthylformamid, 320 ml Triäthylamin und 450 ml Dichlormethan werden vorgelegt. Man leitet unter Rühren und Eiskühlung 1 Mol Phosgen ein. Danach läßt man auf Raumtemperatur kommen und rührt noch 3 Stunden. Innerhalb von 1 Stunde leitet man 40 g Ammoniak (bis zur Sättigung) unter Eiskühlung ein, filtriert und engt ein. Der Rückstand wird im Hochvakuum destilliert.

Kp. 70° C 0,05 Torr
IR: Isonitrilbande: 2160 $cm^{-1}$

Die Herstellung von MAI mit elektronenschiebenden Gruppen im Morpholinorest in analoger Weise durch entsprechende Wahl des Ausgangsamins.

**Patentansprüche:**

1. Verfahren zur Herstellung von Säureamiden durch Umsetzung von Carbonsäure und Amin in Anwesenheit von Isonitril, dadurch gekennzeichnet, daß ein basisches Isonitril mit mindestens einem N-Atom in dem an der Isonitrilgruppe befindlichen Substituenten eingesetzt wird.

2. Verfahren nach Anspruch 1, dadurch gekennzeichnet, daß ein Isonitril eingesetzt wird, in dem das N-Atom im Isonitrilsubstituenten und das C-Atom der Isonitrilfunktion sich in 1,5- oder 1,6-Stellung zueinander befinden.

3. Verfahren nach einem oder mehreren der vorhergehenden Ansprüche, dadurch gekennzeichnet, daß ein Isonitril mit einem N-heterocyclischen Rest verwendet wird.

4. Verfahren nach einem oder mehreren der vorhergehenden Ansprüche, dadurch gekennzeichnet, daß ein Isonitril verwendet wird, das im Substituenten mit einem oder mehreren N-Atomen elektronenschiebende Gruppen enthält.

5. Verfahren nach Ansprüchen 3 oder 4, dadurch gekennzeichnet, daß man als Isonitril Morpholinoethyl-isocyanid, das gegebenenfalls durch eine oder mehrere elektronenschiebende Gruppen substituiert ist, verwendet.

6. Verfahren nach einem oder meheren der vorhergehenden Ansprüche, dadurch gekennzeichnet, daß man zusätzlich N-Hydroxysuccinimid zufügt.

7. Anwendung des Verfahrens nach einem oder mehreren der Ansprüche 1 bis 6 zur Peptidsynthese.

8. Anwendung des Verfahrens nach einem oder mehreren der vorhergehenden Ansprüche 1 bis 6 zur Amidierung von Penicillan- bzw. Cephalosporinderivaten.

9.Morpholino-ethyl-isocyanid.

## Claims

1. A process for the preparation of acid amides by reacting a carboxylic acid and an amine in the presence of an isonitrile, characterized in that a basic isonitrile with at least one nitrogen atom in the substituent present in the isonitrile groups is used.

2. The process according to claim 1, characterized in that an isonitrile is used, wherein the nitrogen atom in the isonitrile substituent and the C-atom of the isonitrile function are in one of the 1.5-and 1.6-positions relative to one another.

3. A process according to one or more of the preceding claims, characterized in that an isonitrile with a N-heterocyclic residue is used.

4. A process according to one or more of the preceding claims, characterized in that an isonitrile is used which contains electron-displacing groups in the substituent with on ore more nitrogen atomes.

5. A process according to claims 3 or 4, characterized in that the isonitrile is morpholino-ethyl isocyanide, optionally substituted by one or more electrondisplacing groups.

6. The process according to one ore more of the preceding claims characterized in that N-hydroxysuccinimide is additionally added.

7. The use of the process according to one or more of claims 1 to 6 in peptide synthesis.

8. The use of the process according to one or more of the preceding claims 1 to 6 for the amidation of penicillane or cephalosporine derivates.

9. Morpholino-ethyl isocyanide.

## Revendications

1. Procédé pour la fabrications d'amides d'acides par réaction d'acide carboxylique et d'amine et d'amine en présence d'isonitrile, caractérisé en ce que l'on utilise un isonitrile basique ayant au moins un atome d'azote dans le substituant lié au groupe isonitrile.

2. Procédé selon la revendication 1, caractérisé en ce que l'on utilise un isonitrile dans lequel l'atome d'azote dans le substituant de l'isonitrile et l'atome de carbone dans la fonction isonitrile se trouvent en position 1,5 ou 1,6 l'un par rapport à l'autre.

3. Procédé selon une ou plusieurs des revendications précédentes, caractérisé en ce que l'on utilise un isonitrile ayant un reste N-hétérocyclique.

4. Procédé selon une ou plusieurs des revendications précédentes, caractérisé en ce que l'on utilise un isonitrile qui contient des groupes repoussant les électrons dans le substituant ayant un ou plusieurs atomes d'azote.

5. Procédé selon les revendications 3 ou 4, caractérisé en ce que l'on utilise comme isonitrile le morpholinoéthylisocyanure qui est éventuellement substitué par un ou plusieurs groupes repoussant les électrons.

6. Procédé selon une ou plusieurs des revendications précédentes, caractérisé en ce que l'on ajoute en outre du N-hydroxysuccinimide.

7. Application du procédé selon une ou plusieurs des revendications 1 à 6 pour la synthèse des peptides.

8. Application du procédé selon une ou plusieurs des revendications précédentes 1 à 6 pour l'amidation des dérivés de pénicillane ou de céphalosporine.

9. Morpholino-éthyl-isocyanure.